# EUROPÄISCHE PATENTSCHRIFT

(11) **EP 0 219 874 B1**
(45) Veröffentlichungstag und Bekanntmachung des Hinweises auf die Patenterteilung: **15.12.1993**
(21) Anmeldenummer: 86114731.2
(22) Anmeldetag: 23.10.1986
(51) Int. Cl.: C07K 3/08, C07K 13/00, C12P 21/02, C12N 9/72

(54) **Verfahren zur Aktivierung von gentechnologisch hergestellten, heterologen, Disulfidbrücken aufweisenden eukaryontischen Proteinen nach Expression in Prokaryonten**
Process for activating recombinant heterologous eucaryotic disulfide-bridged proteins after expression by procaryotes
Procédé d'activation de protéines recombinantes hétérologues et eucaryotiques à pont disulfure après expression dans des procaryotes

(30) Priorität: 23.10.1985 DE 3537708
(43) Veröffentlichungstag der Anmeldung: 29.04.1987
(60) Teilanmeldung: 90109721.2
(73) Patentinhaber: BOEHRINGER MANNHEIM GMBH, 68298 Mannheim (DE)
(72) Erfinder: Rudolph, Rainer, Dr., D-8400 Regensburg (DE); Fischer, Stephan, Dr., D-8120 Weilheim (DE); Mattes, Ralf, Dr., D-8125 Oberhausen (DE)
(74) Vertreter: Weickmann, Heinrich, Dipl.-Ing.

(56) Entgegenhaltungen:
- EP-A- 0 114 506
- WO-A-84/03711
- US-A- 4 530 787
- THROMBOSIS AND HAEMASTASIA, 54(4), 788-791 (1985), D.C. RIJKEN et al.
- METHODS IN ENZYMOLOGY, Band 107, Nr.19, 1984, pp. 305-329, Academic Press Inc. New York, London, T.E. CREIGHTON "Disulfide bond formation in proteins"
- CHEMICAL ABSTRACTS, Band 92, Nr.9, 3. März 1980, Seite 272, Ref. Nr. 71888f, Columbus, Ohio, US, K. MARTINEK et al."Reactivation of "irreversibly" denaturated enzymes"
- BIOCHEMISTRY, Band 21, Nr.19, 1982, Seiten 4734-4740, Am.Chem.Soc. US, Y. KONISHI et al."Regeneration of ribonuclease A from the reduced protein. Rate-limiting steps"
- BIOCHEMISTRY, Band 19, Nr.18, 1980, Seiten 4166-4172, Am. Chem. Soc. US, A. MENEZ et al."Refolding of reduced short neurotoxins : circular dichroism analysis"
- CHEMICAL ABSTRACTS, Band 92, Nr.1, 7. Januar 1980, Seite 233, Ref.Nr, 2302y, Columbus, Ohio, US; G. ZETTLMEISSL et al."Effects of low concentrations of guanidine hydrochloride on the reconstitution of lactic dehydrogenase from pig muscle in vitro"
- HOPPE-SEYLER'S Z. PHYSIOL. CHEM., Band 364, Juli 1983, Seiten 813-820, W. De Gruyter & Co, Berlin, DE; R. RUDOLPH et al."Influence of glutathione on the reactivation of enzymes containing cysteine or cystine"
- CHEMICAL ABSTRACTS, Band 85, Nr.7, 16. August 1976, Seite 244, Ref.Nr. 42985k, Columbus, Ohio, US
- CHEMICAL ABSTRACTS, Band 96, Nr. 13, 29. März 1982, Seite 329, Ref. Nr. 99999z, Columbus, Ohio, US, J. TANG et al."The enhancement of streptokinase activation of plasminogen by nonionic detergents and by serum albumin"
- TEXAS REPORTS ON BIOLOGY AND MEDICINE, Band 41, 1981/82, Seiten 274-279, Univ. of Texas, Galveston, US; J.J. SEDMAK et al."Approaches to the stabilization of interferons"
- CHEMICAL ABSTRACTS, Band 103, Nr. 8, 26. August 1985, Seite 349, Ref. Nr. 59305b, Columbus, Ohio, US
- CHEMICAL ABSTRACTS, Band 91, 1979, Seite 248, Ref.Nr. 34933a, Columbus, Ohio, US; T.W. ODORZYNSKI et al.: "Refolding of the mixed disulfide of bovine trypsinogen and glutathione"
- BIOCHEMISTRY, Band 7, Nr.12, Dezember 1968, Seiten 4247-4254, W.W.-C. CHAN "A method for the complete S suffonation of cysteine residues in proteins
- JOURNAL OF BIOLOGICAL CHEMISTRY, Band 258, Nr.19, 10. Oktober 1983, Seiten 11834-118398, US; J.P. PERRAUDIN et al."Multiple parameter kinetic studies of the oxidative folding of reduced lysozyme"
- CHEMICAL ABSTRACTS, Band 97, 1982, S. 304, Ref. Nr. 122934f, Columbus, Ohio, US; C.T. DUDA et al.: "Refolding of bovine threonine-neochymotrypsinogen"

## Beschreibung

Die Erfindung betrifft ein Verfahren zur Aktivierung von gentechnologisch hergestellten, Disulfidbrücken enthaltenden eukaryontischen Proteinen nach Expression in Prokaryonten.

Bei der Expression von heterologen Proteinen in Prokaryonten bilden diese Proteine in den Wirtszellen oft inaktive, schwer lösliche Aggregate (sog. "refractile bodies"), die außerdem noch mit Proteinen der Wirtszellen verunreinigt sind. Es wird vermutet, daß die Bildung solcher "refractile bodies" eine Folge der bei der Expression entstehenden hohen Proteinkonzentration in der Zelle ist. Es ist bekannt, daß bei der Bildung von großen Enzymmengen in der Zelle die Aggregation der Enzyme zu unlöslichen, hochmolekularen, meist inaktiven Partikeln erfolgt. Bevor solche Proteine, z. B. für therapeutische Zwecke, verwendet werden können, müssen sie demzufolge gereinigt und in ihre aktive Form überführt werden.

Nach bekannten Verfahren kann eine Reaktivierung derartiger, als Aggregate vorliegender Proteine in mehreren Schritten erfolgen (vgl. z. B. R. Jaenicke, FEBS Federation of European Biochemical Societies, Vol. 52 (1979) 187 bis 198; R. Rudolph et al., Biochemistry 18 (1979) 5572 bis 5575):

Im ersten Schritt wird eine Solubilisierung durch Zugabe von starken Denaturierungsmitteln, beispielsweise Guanidin-Hydrochlorid oder Harnstoff in hoher Konzentration oder durch Zugabe von stark sauren Agentien, beispielsweise Glycin/Phosphorsäure-Mischungen erreicht. Als weitere Hilfsstoffe haben sich reduzierende SH-Reagentien (z. B. Dithioerythritol, DTE) und EDTA, beispielsweise bei der Renaturierung von LDH, bewährt. Sofern das Protein durch Proteine der Wirtszelle verunreinigt ist, schließt sich als nächster Schritt eine Reinigung mit an sich bekannten und üblichen Methoden, z. B. Gel oder Ionenaustauschchromatographie, an. Anschließend wird stark verdünnt, damit die Konzentration des Denaturierungsmittels geringer wird. Bei Verwendung von Guanidin-Hydrochlorid wird dabei auf Werte unter 0,5 mol/l verdünnt. Bei Enzymen mit freien SH-Gruppen erwies sich die Zugabe von SH-Gruppen schützenden Agentien als vorteilhaft (vgl. z. B. R. Jaenicke, Journal Polymer Science, Part C 16 (1967) 2143 bis 2160).

In der EP-A-0114506 werden Verfahren zur Isolierung, Reinigung und Reaktïvierung einiger heterologer Expressionsprodukte aus Bakterienkulturen beschrieben; zur Reaktivierung werden die Lösungen der "refractile bodies" in einem starken Denaturierungsmittel a) direkt in eine Lösung in einem schwächeren Denaturierungsmittel überführt, die dann zur Rückbildung von Disulfidbrücken oxydierenden Bedingungen unterworfen wird; b) das Protein sulfoniert, dann in eine Lösung in einem schwachen Denaturierungsmittel überführt und die S-Sulfonatgruppen durch Behandlung mit einem Sulfhydrylreagens in seiner reduzierten und oxydierten Form, z. B. mit GSH/GSSG, in -S-S-Gruppen überführt; oder c) die Lösung in einem schwachen Denaturierungsmittel direkt mit dem Sulfhydryl-Reagens, z. B. mit GSH/GSSG, behandelt. Ein typisches Beispiel, bei dem die oben dargelegten Probleme auftreten, ist t-PA.

In T. W. Odorzynski et al., 1979, The Journal of Biological Chemistry, 254 (10), 4291 - 4295 und in C. T. Duda et al., 1982, The Journal of Biological Chemistry, 257 (16), 9866 - 9871 werden verfahren zur Reaktivierung von natürlichen Proteinen, wie z. B. Trypsinogen, beschrieben.

Die Hauptkomponente der Proteinmatrix von geronnenem Blut ist polymeres Fibrin. Diese Proteinmatrix wird durch Plasmin gelöst, das aus Plasminogen über Aktivierung durch die sogenannten Plasminogen-Aktivatoren gebildet wird, z. B durch t-PA (Gewebs-PlasminogenAktivator, tissue-type plasminogen activator). Die enzymatische Aktivität von natürlichem oder aus Eukaryonten gentechnologisch gewonnenem t-PA (katalytische Aktivierung von Plasminogen zu Plasmin) ist in Abwesenheit von Fibrin oder Fibrinspaltprodukten (FSP) sehr gering, kann aber in Gegenwart dieser Stimulatoren wesentlich gesteigert werden (um mehr als den Faktor 10). Diese sogenannte Stimulierbarkeit der Aktivität ist ein entscheidender Vorzug von t-PA gegenüber anderen bekannten Plasminogenaktivatoren, wie Urokinase oder Streptokinase (vgl. z. B. M. Hoylaerts et al., J. Biol. Chem. 257 (1982) 2912 bis 2019; Nieuwenhiuzen et al., Biochemica et Biophysica Acta 755 (1983) 531 bis 533). Der Faktor der Stimulierbarkeit mit BrCN-Spaltprodukten wird daher in der Literatur verschiedentlich angegeben und mit bis zu 35 beziffert.

Ein t-PA-artiges, nicht glycosyliertes Produkt wird auch in genetisch manipulierten Prokaryonten (nach Einschleusen der c-DNA) gebildet; einem solchen Produkt kommt aber nicht die Stimulierbarkeit der Aktivität eines t-PA aus Eukaryonten zu. Denkbar ist, daß dies darauf zurückzuführen ist, daß die Redoxbedingungen in der Prokaryontenzelle in solcher Weise von der Eukaryontenzelle, aus der das Gen stammt, verschieden sind, daß von Anfang an ein nicht aktives Produkt gebildet wird, was beispielsweise darauf zurückzuführen sein könnte, daß die zahlreichen SS-Brücken, die das natürliche aktive Molekül enthält, in falscher Weise verknüpft oder gar nicht gebildet sind. Für den therapeutischen Einsatz von t-PA ist aber nicht nur die enzymatische Aktivität als solche erforderlich, sondern außerdem auch seine Stimulierbarkeit. Auf die Tatsache, daß die Prokaryontenzelle vermutlich nicht die richtigen Bedingungen schafft, um die Aktivität von Eukaryontenproteinen in der richtigen Weise auszubilden, wird für andere Substanzen in The EMBO Journal 4, Nr. 3 (1985) 775 bis 780 hingewiesen.

Nach der EP-A-0093639 werden zur Reaktivierung von t-PA die aus E. coli erhaltenen Zellpellets in 6 mol/l Guanidin-Hydrochlorid suspendiert, mit Ultraschall behandelt, inkubierr und anschließend vier Stunden gegen eine Lösung aus Tris-HCl (pH = 8,0), Natriumchlorid, EDTA und Tween 80 dialysiert. Nach Dialyse wird zentrifugiert, wobei im Überstand die Plasminogenaktivator-Aktivität zu finden ist. Auf diese Weise renaturierter t-PA ist zwar proteolytisch aktiv, zeigt jedoch keine meßbare Stimulierbarkeit durch BrCN-Spaltprodukte (BrCN-FSP) von Fibrin, gemäß dem in J. H. Verheijen, Thromb. Haemostas., 48, (3), 260 - 269 (1982) beschriebenen Verfahren.

Für die Reaktivierung von denaturierten Proteinen ist aus dem Stand der Technik kein allgemein anwendbares Verfahren bekannt; dies gilt ganz besonders für t-PA, weil das native Protein eine sehr komplexe Struktur besitzt; es enthält eine freie Thiolgruppe und 17 SS-Brücken, die theoretisch auf 2,2 x 10²⁰ verschiedene Weisen verknüpft werden können, wobei nur eine Struktur dem nativen Zustand entspricht. Die aus dem Stand der Technik bekannten Verfahren zur Reaktivierung von t-PA führen zwar zu einem proteolytisch aktiven t-PA, der aber keine meßbare Stimulierbarkeit zeigt; ein Aktivierungsverfahren, welches zu stimulierbarem t-PA führt, ist nicht bekannt.

Aufgabe der vorliegenden Erfindung ist deshalb die Bereitstellung eines Verfahrens zur vollständigen Aktivierung von gentechnologisch hergestellten, heterologen, Disulfidbrücken enthaltenden eukaryontischen Proteinen nach Expression in Prokaryonten; diese Aufgabe wird mit dem Gegenstand der vorliegenden Erfindung gelöst.

Gegenstand der Erfindung ist ein Verfahren zur Aktivierung von durch Expression in Prokaryonten gentechnologisch hergestellten, heterologen, Disulfidbrücken aufweisenden eukaryontischen Proteinen durch
a) Aufschließen der Prokaryontenzellen,
b) Solubilisierung der eukaryontischen Proteine unter denaturierenden und reduzierenden Bedingungen,
c) Abtrennung der Reduktions-/Denaturierungsmittel
d) Reaktivierung unter oxidierenden Bedingungen durch
e) Überführung der Thiolgruppen der solubilisierten Proteine in die gemischten Disulfide von Protein und Glutathion durch Zugabe von GSSG unter denaturierenden Bedingungen,
f) Bildung von aktivem Protein aus den gemischten Disulfiden bei einer GSH-Konzentration von 0,5 bis 5 mmol/l, einem pH-Wert von 7 bis 10,5 und in Gegenwart einer nicht denaturierenden Konzentration eines Denaturierungsmittels.

Bevorzugte Ausgestaltungen dieses Verfahrens sind Gegenstand der Unteransprüche.

Als Denaturierungsmittel kann in der Regel ein für Aktivierungen unter oxidierenden Bedingungen üblicherweise verwendetes Denaturierungsmittel oder Arginin eingesetzt werden; bevorzugt wird unter den bekannten Denaturierungsmitteln Guanidin-Hydrochlorid oder Harnstoff oder dessen Derivate verwendet. Außerdem hat sich Arginin als geeignet erwiesen. Ferner können Gemische dieser Denaturierungsmittel verwendet werden. Vorzugsweise wird diese Aktivierungsstufe auch in Gegenwart eines Fremdproteins durchgeführt; als solches eignet sich in der Regel jedes Fremdprotein, solange es nicht proteolytisch wirksam ist; vorzugsweise wird Rinderserumalbumin (BSA), verwendet, z.B. in einer Menge von 1 bis 3 mg/ml. Der Zusatz von BSA bewirkt eine leichte Erhöhung der Ausbeute und Stabilisierung des Proteins (wahrscheinlich durch Schutz vor Oberflächendenaturierung und/oder proteolytischem Abbau).

Die übrigen Verfahrensbedingungen können den für Reaktivierungsstufen aus dem Stand der Technik bekannten und üblichen Bedingungen entsprechen. Die Dauer der Aktivierung (Inkubation) beträgt vorzugsweise 20 bis 48 Stunden bei Raumtemperatur. Bei einer längeren Inkubation (48 Stunden) unter Reoxidationsbedingungen nimmt die Stimulierbarkeit durch CNBr-FSP in der Regel ab. Die Aktivierungsstufe wird vorzugsweise in Gegenwart von EDTA durchgeführt, wobei die zweckmäßigste Konzentration ca. 1 mmol/l EDTA beträgt.

Die der Aktivierungsstufe (Reoxidation/Aktivierung) vorausgehenden und nachfolgenden Verfahrensschritte, wie Zellaufschluß, Solubilisierung (Solubilisierung/Reduktion) und gegebenenfalls eine oder mehrere der Aktivierungsstufe vorausgehende und/oder nachfolgende Reinigungsoperationen können nach aus dem Stand der Technik, z.B. aus der EP-A-0114506, EP-A-0093619, für derartige Verfahren bekannten und üblichen Methoden durchgeführt werden; für ein im Hinblick auf Ausbeute und Aktivierung optimales Ergebnis kann es aber zweckmäßig sein, einzelne oder alle Verfahrensschritte unter Berücksichtigung einer oder mehrerer der hier erläuterten Verfahrensausgestaltungen durchzuführen. Die Expression wird in Prokaryonten, vorzugsweise in P. putida, und insbesondere in E. coli, durchgeführt. Das erfindungsgemäße Verfahren ist jedoch genauso geeignet, wenn in anderen Prokaryonten (z.B. Bacilli) exprimiert wird.

Der Zellaufschluß kann durch hierfür übliche Methoden durchgeführt werden, z.B. mittels Ultraschall, Hochdruckdispersion oder Lysozym; er wird vorzugsweise in einer zur Einstellung eines neutralen bis schwach sauren pH-Wertes geeigneten Pufferlösung als Suspensionsmedium durchgeführt, wie z.B. in 0,1 mol/l Tris-HCl. Nach dem Zellaufschluß werden die unlöslichen Bestandteile ("refractile bodies") in beliebiger Weise abgetrennt, vorzugsweise durch Abzentrifugieren bei höheren g-Zahlen und längerer Zentrifugationszeit oder durch Filtration. Nach dem Waschen mit Agentien, die nicht stören, fremde Zellproteine jedoch möglichst lösen, z.B. Wasser, Phosphat-Pufferlösung, gegebenenfalls unter Zusatz milder Detergentien wie Triton, wird der Niederschlag (Pellet) der Solubilisierung (Solubilisierung/Reduktion) unterworfen. Die Solubilisierung erfolgt vorzugsweise im alkalischen pH-Bereich, insbesondere bei pH = 8,6 ± 0,4 und in Gegenwart eines Reduktionsmittels aus der Mercaptangruppe und eines Denaturierungsmittels.

Als Denaturierungsmittel können die für Solubilisierungen aus dem Stand der Technik, z.B. aus der EP-A-0114506, bekannten und üblichen Denaturierungsmittel verwendet werden, und insbesondere Guanidin-Hydrochlorid oder Harnstoff. Die Konzentration an Guanidin-Hydrochlorid beträgt zweckmäßigerweise ca. 6 mol/l, die von Harnstoff ca. 8 mol/l. Ebenso können Verbindungen der allgemeinen Formel I eingesetzt werden.

Als Reduktionsmittel aus der Mercaptangruppe kann z.B. reduziertes Glutathion (GSH) oder 2-Mercaptoäthanol verwendet werden, z.B. in einer Konzentration von ca. 50 bis 400 mmol/l und/oder insbesondere DTE (Dithioerythritol) bzw. DTT (Dithiothreitol), z.B. in einer Konzentration von ca. 80 bis 400 mmol/l. Die Solubilisierung erfolgt zweckmäßigerweise bei Raumtemperatur während einer Dauer (Inkubation) von 1 bis mehreren Stunden, vorzugsweise von zwei Stunden. Zur Verhinderung der Aufoxidation des Reduktionsmittels durch Luftsauerstoff kann es auch zweckmäßig sein, EDTA zuzusetzen. Neben der Solubilisierung/Reduktion hat die Solubilisierungsstufe auch einen Reinigungseffekt, da ein Großteil der Fremdproteine nicht in Lösung geht.

Nach der Solubilisierung erfolgt die Abtrennung der Reduktions- und der Denaturierungsmittel, eine unspezifische Reoxidation kann durch Zusatz eines Reduktionsmittels (z.B. 2-Mercaptoäthanol) oder durch pH-Werte 4,5 verhindert werden (vgl. z.B. R. Rudolph, Biochem. Soc. Transactions 13 (1985) 308 bis 311). Die Abtrennung der Denaturierungs/Reduktionsmittel erfolgt durch Entsalzen über Sephadex G 25 in 0,01 mol/l HCl bzw. 0,1 mol/l Essigsäure. Die Abtrennung der Denaturierungs-/Reduktionsmittel ist alternativ durch Dialyse gegen die gleichen Lösungen möglich.

Ein weiterer Reinigungsschritt kann sich an die Reaktivierungsstufe anschließen; eine solche Reinigung erfolgt in der Regel mittels Dialyse, oder auch einer anschließenden Isolierung des aktivierten Proteins, beispielsweise durch Affinitätschromatographie, beispielsweise über Lys-Sepharose.

Die Bildung der gemischten Disulfide der gentechnologisch hergestellten, heterologen, Disulfidbrücken enthaltenden eukaryontischen Proteine mit Glutathion (im folgenden abgekürzt t-PASSG) erleichtert sowohl die Abtrennung von Fremdproteinen im denaturierten Zustand als auch die weitere Reinigung des nativen Proteins. Eine Reinigung nach Modifizierung der Thiolgruppen hat den Vorteil, daß das Protein geschützt gegen Luftoxidation und damit in einem größeren pH-Bereich stabil ist und eine Veränderung der Nettoladung die Reinigung erleichtert. Insbesondere kann durch Ionenaustauscherbehandlung eine Abtrennung vom nicht modifizierten Protein vorteilhaft durchgeführt werden.

Zur Bildung der gemischten Disulfide wird das dialysierte, reduzierte, von Denaturierungs- und Reduktionsmitteln gereinigte Protein mit einer ein Denaturierungsmittel enthaltenden, verdünnten, z.B. 0,2 mol/l, Lösung von GSSG inkubiert. Die Aktivierung erfolgt nach Abtrennung des Denaturierungs- und des Oxidationsmittels bei einem pH-Wert von 7 bis 10,5 einer GSH-Konzentration von 0,5 bis 5 mmol/l und mit einer nicht denaturierenden Konzentration des Denaturierungsmittels.

Das pH-Optimum liegt bei 8,5, die Ausbeute ist etwa doppelt so hoch wie bei einer Reaktivierung mit GSH/GSSG ohne gesonderte Bildung der gemischten Disulfide und das aktivierte Protein ist im Renaturierungspuffer über längere Zeit stabil.

Erfindungsgemäß gelingt es, t-PA aus Prokaryonten so zu aktivieren, daß nicht nur eine Aktivierung der normalen biologischen Aktivität erreicht, sondern darüberhinaus auch eine Stimulierbarkeit im oben definierten Sinne erreicht wird, welche die Stimulierbarkeit des nativen t-PA weit übersteigt und größer als Faktor 10 ist, ja sogar Faktor 50 übersteigen kann.

Ein weiteres eukaryontisches Protein, das erfindungsgemäß nach Expression in Prokaryonten aktiviert werden kann, ist β-Interferon.

Das nachfolgende Beispiel erläutert die Erfindung näher, ohne sie darauf zu beschränken. Wenn nicht anders angegeben, beziehen sich Prozentangaben auf Gewichtsprozent, und Temperaturangaben auf Grad Celsius.

### Beispiel

a) Präparation der "refractile bodies"
100 g E.coli Zellfeuchtmasse, aufgenommen in 1,5 l, 0,1 mol/l Tris/HCl (pH 6,5) und 20 mmol/l EDTA wurden homogenisiert (Ultra-Turrax, 10 Sek.) und 0,25 mg/ml Lysozym zugegeben. Nach 30 Min. Inkubation bei Raumtemperatur wurde erneut homogenisiert und auf 3°C abgekühlt. Der Zellaufschluß wurde durch Hochdruckdispersion (550 kg/cm²) erreicht. Anschließend wurde mit 300 ml 0,1 mol/l Tris/HCl (pH 6,5) und 20 mmol/l EDTA nachgespült. Nach Zentrifugation (2 Std. bei 27.000 g, 4°C) wurde das Pellet in 1,3 l 0,1 mol/l Tris/HCl (pH 6,5), 20 mmol/l EDTA und 2,5 % Triton-x-100 aufgenommen und homogenisiert. Nach erneuter Zentrifugation (30 Min. bei 27.000 g, 4°C) wurde das Pellet in 1,3 l 0,1 mol/l Tris/HCl (pH 6,5), 20 mmol/l EDTA und 0,5 % Triton-x-100 aufgenommen und homogenisiert. Abwechselnde Zentrifugation (30 Min bei 27.000 g, 4°C) und Homogenisation des Pellets in 1 l 0,1 mol/l Tris/HCl (pH 6,5) und 20 mmol/l EDTA wurde noch dreimal durchgeführt.
Der t-PA-Gehalt der "refractile bodies" Präparationen wurde durch SDS-PAGE, Identifizierung der t-PA-Banden durch "Western-blotting" und densitometrische Analyse quantifiziert. Die "refractile bodies" zeigen bei SDS-PAGE und "Western-blotting" eine starke t-PA-Bande mit einem Molekulargewicht von ca. 60 kDa. Der t-PA-Anteil am Gesamtproteingehalt der "refractile bodies" beträgt ca. 21 %.
b) Aktivierung von t-PA über die gemischten Disulfide von t-PA und Glutathion.
Die verwendeten "refractile bodies" wurden nach a) erhalten. Die Reduktion der "refractile bodies" wurde durch 2 Stunden Inkubation bei Raumtemperatur in 0,1 mol/l Tris/HCl, pH 8,6, 1 mmol/l EDTA, 6 mol/l Gdn·HCl, 0,2 mol/l DTE bei einer Proteinkonzentration von etwa 1mg/ml durchgeführt.
Das gegen 0,01 mol/l HCl dialysierte, reduzierte Protein wurde im Verhältnis 1:1 mit 0,1 mol/l Tris, pH 9,3, 9 mol/l Harnstoff und 0,2 mol/l GSSG verdünnt und 5 Stunden bei Raumtemperatur inkubiert.
Nach Ansäuern mit konz. HCl auf pH 3 folgte Dialyse gegen 0,01 mol/l HCl bei 4°C. Nach der Dialyse betrug die Gesamtproteinkonzentration 0,33 mg/ml. Mit dem so präparierten t-PASSG wurden die optimalen Reaktivierungsbedingungen bestimmt.
c) pH-Optimum der Aktivierung von t-PASSG
Hier, wie in den folgenden Optimierungsexperimenten wurde (1) kein GSSG verwendet und (2) die Aktivierung nach 17 Stunden Inkubation bei Raumtemperatur bestimmt. Aktivierung erfolgte durch eine 1:100 Verdünnung in 0,1 mol/l Tris, 1 mmol/l EDTA, 0,5 mol/l L-Arginin, 1 mg/ml BSA und 2 mmol/l GSH bei Variation des pH-Wertes.

| pH | Ausbeute (%) | Stimulierbarkeit |
|---|---|---|
| 6 | 0,04 | 3,3 |
| 6,5 | 0,37 | 9,5 |
| 7 | 1,35 | 11,4 |
| 7,5 | 5,66 | 7,1 |
| 8 | 7,32 | 8,2 |
| 8,5 | 8,65 | 7,0 |
| 9 | 8,59 | 8,7 |
| 9,5 | 8,32 | 11,7 |
| 10 | 6,15 | 12,5 |
| 10,5 | 3,07 | 11,2 |

Die Ausbeute wurde in % aktiver t-PA bezogen auf eingesetzte Proteinmenge bestimmt.
d) Reproduzierbarkeit der Ergebnisse der Aktivierung von t-PASSG
Bei identischen Aktivierungsbedingungen werden bei verschiedenen Meßreihen unterschiedliche Ausbeuten beobachtet, die u.a. durch Schwankungen des Standard-t-PA's bedingt sind. Zur Verdeutlichung dieser Fehlerbreite sind alle Aktivierungsdaten nach 1:100 bzw. 1:200 Verdünnung in 0,1 mol/l Tris/HCl, pH 8,5, 1 mmol/l EDTA, 0,5 mol/l L-Arginin, 1 mg/ml BSA und 2 mmol/l GSH zusammengefaßt.

| Versuch | Ausbeute (%) | Stimulierbarkeit |
|---|---|---|
| 1 | 8,65 | 7,0 |
| 2 | 4,47 | 9,3 |
| 3 | 4,49 | 9,7 |
| 4 | 8,50 | 6,5 |
| 5 | 3,45 | 17,2 |
| 6 | 4,32 | 8,3 |
| 7 | 3,29 | 14,0 |
| 8 | 3,54 | 13,4 |
| 9 | 5,07 | 16,4 |
| Mittelwert | 5,1 +/- 1,9 | 11,3 +/- 3,8 |

e) Stabilität des aktivierten Proteins
Aktivierung erfolgte in dem genannten Beispiel durch eine 1:200 Verdünnung in 0,1 mol/l Tris/HCl, 1 mmol/l EDTA, 0,5 mol/l L-Arginin, 1 mg/ml BSA und 2 mmol/l GSH.

| Zeit (h) | pH 9,5 | |
|---|---|---|
| | Ausb. (%) | Stim. |
| 1 | 0 | - |
| 6 | 0,89 | 15,5 |
| 23 | 2,43 | 23,1 |
| 47 | 2,83 | 23,6 |
| 71 | 2,62 | 21,5 |
| 215 | 2,21 | 22,6 |
| 239 | 2,28 | 14,3 |

## Patentansprüche

1. Verfahren zur Aktivierung von durch Expression in Prokaryonten gentechnologisch hergestellten, heterologen, Disulfidbrücken aufweisenden eukaryontischen Proteinen durch
a) Aufschließen der Prokaryontenzellen,
b) Solubilisierung der eukaryontischen Proteine unter denaturierenden und reduzierenden Bedingungen,
c) Abtrennung der Reduktions-/Denaturierungsmittel,
d) Reaktivierung unter oxidierenden Bedingungen durch
e) Überführung der Thiolgruppen der solubilisierten Proteine in die gemischten Disulfide von Protein und Glutathion durch Zugabe von GSSG unter denaturierenden Bedingungen,
f) Bildung von aktivem Protein aus den gemischten Disulfiden bei einer GSH-Konzentration von 0,5 bis 5 mmol/l, einem pH-Wert von 7 bis 10,5 und in Gegenwart einer nicht denaturierenden Konzentration eines Denaturierungsmittels.

2. Verfahren nach Anspruch 1,
**dadurch gekennzeichnet,** daß man die Expression in E. coli oder P. putida durchführt.

3. Verfahren nach Anspruch 1 oder 2,
**dadurch gekennzeichnet,** daß man in der Reaktivierungsstufe als Denaturierungsmittel Arginin, Guanidinhydrochlorid und/oder wenigstens eine Verbindung der allgemeinen Formel R₂-CO-NRR₁ (I), in der R und R₁H oder Alkyl mit 1 bis 4 C-Atomen und R₂H oder NHR₁ oder Alkyl mit 1 bis 3 C-Atomen bedeutet, verwendet.

4. Verfahren nach Anspruch 3, **dadurch gekennzeichnet,** daß die Konzentration an Arginin und/oder Guanidinhydrochlorid 0,1 bis 1,0 mol/l, insbesondere 0,25 bis 0,8 mol/l beträgt.

5. Verfahren nach Anspruch 3, **dadurch gekennzeichnet,** daß die Konzentration der Verbindung der allgemeinen Formel I 0,5 bis 4 mol/l, insbesondere 1 bis 3.5 Mol/l beträgt.

6. Verfahren nach einem der vorhergehenden Ansprüche, **dadurch gekennzeichnet,** daß man in der Stufe der Reaktivierung in Gegenwart eines nicht proteolytisch wirksamen Proteins, insbesondere in Gegenwart von Rinderserumalbumin, arbeitet.

7. Verfahren nach einem der vorhergehenden Ansprüche, **dadurch gekennzeichnet,** daß man den Zellaufschluß mittels Ultraschall, Hochdruckdispersion oder Lysozym durchführt.

8. Verfahren nach Anspruch 7, **dadurch gekennzeichnet,** daß man den Aufschluß in einer verdünnten wässrigen Pufferlösung, insbesondere in 0,1 mol/l Tris, bei einem neutralen bis schwach sauren pH-Wert durchführt.

9. Verfahren nach einem der vorhergehenden Ansprüche, **dadurch gekennzeichnet,** daß man nach dem Zellaufschluß die unlöslichen Bestandteile abtrennt.

10. Verfahren nach einem der vorhergehenden Ansprüche, **dadurch gekennzeichnet,** daß man in der Stufe der Solubilisierung bei einem alkalischen pH-Wert in Gegenwart eines Reduktionsmittels aus der Mercaptogruppe und in Gegenwart eines Denaturierungsmittels arbeitet.

11. Verfahren nach Anspruch 10, **dadurch gekennzeichnet,** daß man in Gegenwart von Guanidinhydrochlorid und/oder Verbindung der allgemeinen Formel I als Denaturierungsmittel arbeitet.

12. Verfahren nach Anspruch 11, **dadurch gekennzeichnet,** daß die Konzentration an Guanidinhydrochlorid 6 mol/l, die der Verbindung der allgemeinen Formel I 8 mol/l, beträgt.

13. Verfahren nach einem der Ansprüche 10 bis 12, **dadurch gekennzeichnet,** daß man in Gegenwart von DTE, β-Mercaptoethanol, Cystein oder GSH arbeitet.

14. Verfahren nach einem der vorhergehenden Ansprüche, **dadurch gekennzeichnet,** daß man Reinigung und Abtrennung von Reduktions-, Oxidations- oder Denaturierungsmitteln mittels sterischer Ausschlußchromatographie oder Dialyse durchführt.

15. Verfahren nach einem der vorhergehenden Ansprüche, **dadurch gekennzeichnet,** daß man nach der Stufe der Reaktivierung eine Reinigungsstufe mittels Dialyse durchführt.

16. Verfahren nach einem der Ansprüche von 1 bis 15, **dadurch gekennzeichnet,** daß man als gentechnologisch hergestelltes eukaryontisches Protein t-PA verwendet.

17. Stimulierbarer nicht glycosylierter tPA, erhältlich nach dem Verfahren gemäß einem der Ansprüche 1 bis 16.

18. Verfahren nach Anspruch 6, **dadurch gekennzeichnet,** daß man das gemischte Disulfid von Protein und Glutathion durch Ionenaustauscherbehandlung vom nichtmodifizierten Protein abtrennt.

## Claims

1. Process for the activation of heterologous, disulphide bridge-containing eukaryotic proteins produced gene-technologically by expression in prokaryotes by
a) digestion of the prokaryote cells,
b) solubilisation of the eukaryotic proteins under denaturing and reducing conditions,
c) separation of the reducing/denaturing agents,
d) reactivation under oxidising conditions by
e) conversion of the thiol groups of the solubilised proteins into the mixed disulphides of protein and glutathione by addition of GSSG under denaturing conditions,
f) formation of active protein from the mixed disulphides at a GSH concentration of 0.5 to 5 mmol/l, a pH value of 7 to 10.5 and in the presence of a non-denaturing concentration of a denaturing agent.

2. Process according to claim 1, characterised in that one carries out the expression in E. coli or P. putida.

3. Process according to claim 1 or 2, characterised in that, in the reactivation step, as denaturing agent, one uses arginine, guanidine hydrochloride and/or at least one compound of the general formula R₂-CO-NRR₁ (I), in which R and R₁ signify H or alkyl with 4 C-atoms and R₂ signifies H or NHR₁ or alkyl with 1 to 3 C-atoms.

4. Process according to claim 3, characterised in that the concentration of arginine and/or guanidine hydrochloride amounts to 0.1 to 1.0 mol/l, especially 0.25 to 0.8 mol/l.

5. Process according to claim 3, characterised in that the concentration of the compound of the general formula I amounts to 0.5 to 4 mol/l, especially 1 to 3.5 mol/l.

6. Process according to one of the preceding claims, characterised in that, in the step of reactivation, one works in the presence of a non-proteolytically effective protein, especially in the presence of bovine serum albumin.

7. Process according to one of the preceding claims, characterised in that one carries out the cell digestion by means of ultrasonics, high pressure dispersion or lysozyme.

8. Process according to claim 7, characterised in that one carries out the digestion in a dilute aqueous buffer solution, especially in 0.1 mol/l tris, at a neutral to weakly acidic pH value.

9. Process according to one of the preceding claims, characterised in that, after the cell digestion, one separates off the insoluble components.

10. Process according to one of the preceding claims, characterised in that, in the step of solubilisation, one works at an alkaline pH value in the presence of a reducing agent of the mercapto group and in the presence of a denaturing agent.

11. Process according to claim 10, characterised in that one works in the presence of guanidine hydrochloride and/or of the general formula I as denaturing agent.

12. Process according to claim 11, characterised in that the concentration of guanidine hydrochloride amounts to 6 mol/l, that of the compound of the general formula I to 8 mol/l.

13. Process according to one of claims 10 to 12, characterised that one works in the presence of DTE, β-mercaptoethanol, cysteine or GSH.

14. Process according to one of the preceding claims, characterised in that one carries out purification and separation of reducing, oxidising and denaturing agents by means of steric exclusion chromatography or dialysis.

15. Process according to one of the preceding claims, characterised in that, after the step of reactivation, one carries out a purification step by means of dialysis.

16. Process according to one of claims 1 to 15, characterised in that one uses t-PA as gene-technologically produced eukaryotic protein.

17. Stimulatable, non-glycosilated t-PA, obtainable according to the process according to one of claims 1 to 16.

18. Process according to claim 6, characterised in that one separates the mixed disulphide of protein and glutathione from the non-modified protein by ion exchanger treatment.

## Revendications

1. Procédé d'activation de protéines eucaryotiques hétérologues, comportant des ponts disulfures, préparées par génie génétique par expression dans des procaryotes, par
a) lyse des cellules procaryotiques,
b) solubilisation des protéines eucaryotiques dans des conditions dénaturantes et réductrices,
c) séparation des agents réducteurs/dénaturants,
d) réactivation dans des conditions oxydantes par
e) conversion des groupes thiols des protéines solubilisées en les disulfures mixtes de protéine et de glutathion par addition de GSSG dans des conditions dénaturantes,
f) formation de protéine active à partir des disulfures mixtes à une concentration en GSH de 0,5 à 5 mmol/l, un pH de 7 à 10,5 et en présence d'une concentration non dénaturante d'un agent dénaturant.

2. Procédé selon la revendication 1, caractérisé en ce que l'on accomplit l'expression dans E. coli ou P. putida.

3. Procédé selon la revendication 1 ou 2, caractérisé en ce que l'on utilise comme agent dénaturant dans l'étape de réactivation l'arginine, le chlorhydrate de guanidine et/ou au moins un composé de formule générale R₂-CO-NRR₁ (I), dans laquelle R et R₁ signifient H ou alkyle de 1 à 4 atomes de carbone et R₂ signifie H ou NHR₁ ou alkyle de 1 à 3 atomes de carbone.

4. Procédé selon la revendication 3, caractérisé en ce que la concentration en arginine et/ou en chlorhydrate de guanidine est de 0,1 à 1,0 mol/l, en particulier de 0,25 à 0,8 mol/l.

5. Procédé selon la revendication 3, caractérisé en ce que la concentration du composé de formule générale I est de 0,5 à 4 mol/l, en particulier de 1 à 3,5 mol/l.

6. Procédé selon l'une des revendications précédentes, caractérisé en ce que, dans l'étape de réactivation, on opère en présence d'une protéine non protéolytique, en particulier en présence de sérumalbumine bovine.

7. Procédé selon l'une des revendications précédentes, caractérisé en ce que l'on accomplit la lyse des cellules au moyen d'ultrasons, par dispersion sous haute pression ou à l'aide de lysozyme.

8. Procédé selon la revendication 7, caractérisé en ce que l'on accomplit la lyse dans une solution tampon aqueuse diluée, en particulier dans du tris à 0,1 mol/l, à pH neutre à faiblement acide.

9. Procédé selon l'une des revendications précédentes, caractérisé en ce que l'on sépare les constituants insolubles après la lyse des cellules.

10. Procédé selon l'une des revendications précédentes, caractérisé en ce que, dans l'étape de solubilisation, on opère à pH alcalin en présence d'un agent réducteur du groupe des mercaptans et en présence d'un agent dénaturant.

11. Procédé selon la revendication 10, caractérisé en ce que l'on opère en présence de chlorhydrate de guanidine et/ou d'un composé de formule générale I en tant qu'agent dénaturant.

12. Procédé selon la revendication 11, caractérisé en ce que la concentration du chlorhydrate de guanidine est de 6 mol/l et la concentration du composé de formule générale I est de 8 mol/l.

13. Procédé selon l'une des revendications 10 à 12, caractérisé en ce que l'on opère en présence de DTE, de β-mercaptoéthanol, de cystéine ou de GSH.

14. Procédé selon l'une des revendications précédentes, caractérisé en ce que l'on accomplit la purification et la séparation des agents réducteurs, oxydants ou dénaturants par chromatographie d'exclusion stérique ou par dialyse.

15. Procédé selon l'une des revendications précédentes, caractérisé en ce que l'on réalise une étape de purification par dialyse après l'étape de réactivation.

16. Procédé selon l'une des revendications 1 à 15, caractérisé en ce que l'on utilise le t-PA comme protéine eucaryotique préparée par génie génétique.

17. T-PA non glycosylé stimulable qui peut être obtenu par le procédé selon l'une des revendications 1 à 16.

18. Procédé selon la revendication 6, caractérisé en ce que l'on sépare par traitement sur échangeur d'ions le disulfure mixte de protéine et de glutathion de la protéine non modifiée.
